Europäisches Patentamt

European Patent Office

Office européen des brevets

(19)

(11) Veröffentlichungsnummer: **0 068 042**
**A1**

(12) # EUROPÄISCHE PATENTANMELDUNG

(21) Anmeldenummer: **81105110.1**

(22) Anmeldetag: **01.07.81**

(51) Int. Cl.³: **A 61 K 35/26**
**// (A61K35/26, 35/23)**

(43) Veröffentlichungstag der Anmeldung: **05.01.83**
**Patentblatt 83/1**

(71) Anmelder: **Selzer, Hans, Dr., Schönmünzach,**
**D-7292 Baiersbronn 9 (DE)**

(72) Erfinder: **Selzer, Hans, Dr., Schönmünzach,**
**D-7292 Baiersbronn 9 (DE)**

(74) Vertreter: **Freiherr von Welser, Hubert, Danziger**
**Strasse 15, D-8000 München 40 (DE)**

(84) Benannte Vertragsstaaten: **AT CH FR GB IT LI SE**

(54) **Heilmittel aus Lymphe von Säugetieren.**

(57) Heilmittel, das aus Lymphe von Säugetieren gewonnen ist und zur Bekämpfung von Injektionen, Krebs- und Autoimmunerkrankungen sowie zur Beseitigung von gefährlichen Blutergüssen bestimmt ist. Das Heilmittel ist aus dem Ductus thoracicus des Spendertieres gewonnen und durch einen Filter mit der Porengröße von 10 μm unter Druck von 0,3 bis 0,5 bar gewonnen und es können ihm Nebennieren- und Nebennierenrindenextrakte zugesetzt sein.

EP 0 068 042 A1

- I -

Die Erfindung betrifft ein Heilmittel aus Lymphe
von Säugetieren. Ein solches Heilmittel hat den
Zweck, die Immunantwort des Patienten gegen
Antigen zu verstärken. Bekannt ist, aus lymphatischen
Organen von Säugetieren Extrakte zu gewinnen, z. B.
aus dem Thymus oder der Milz (z.B. DE-AS 2 110 436),
es werden dabei aber nicht die lebenden Lymphzellen
benutzt, ebenso wenig wie bei der Serumtherapie,
die sich allein auf die Verwendung der von dem Lymphsystem des Spendertieres erzeugten Antikörper beschränkt. Die Abtrennung der Lymphzellen und anderer
zellulärer und großmolekularer Bestandteile begründete
sich in der Gefahr von Immunreaktionen oder Überempfindlichkeitsreaktionen des Abwehrsystems des
Empfängers gegen die ihm übertragenen Lymphbestandteile.

In US-PS 3 719 182 und DE-OS 2 416 842 wird ein Verfahren beschrieben, mittels einer an Patienten im
Milchbrustgang angelegten Fistel über längere Zeitabschnitte nach Möglichkeit die gesamte Lymphproduktion
zu entnehmen, daraus die Antikörper und Lymphzellen
durch Zentrifugieren abzuscheiden und die Lymphflüssigkeit und die Lymphzellen unter Umständen nach Reinigung
dem Körper des Patienten zurückzugeben. Auch dieses
Verfahren zielt auf die Vermehrung der vom Lymphsystem
des Patienten selbst erzeugten Antikörper, insbesondere
jener, die für die angestrebte Therapie spezifiziert
sind. Die Zentrifugierung der Lymphzellen und deren
weitere Behandlung bringt die Gefahr ihrer Schädigung
mit sich.

- 2 -

Im Gegensatz zu dieser auf spezifische Antigene
zielenden Heilverfahren und Mittel ist es Aufgabe
der Erfindung, lebende und völlig intakte Lymphzellen von Spendertieren zu gewinnen, die dem
menschlichen Empfänger durch Injektion in die erkrankten Bereiche oder intravaskulär zugeführt
werden können, ohne daß dadurch Immunreaktionen oder
Überempfindlichkeitsreaktionen des Immunsystems
des Empfängers gegen das übertragene Material hervorgerufen werden. Dabei ist es wichtig, auch im
wesentlichen Umfang nicht sensibilisierte Lymphzellen
zu übertragen, die dann erst im Immunsystem des
Empfängers auf die nicht erkenn- oder isolierbaren
krankhaften Prozesse sensibilisiert werden, um dort
Funktionen der Immunabwehr, auch solche noch nicht
aufgeklärter Art zu übernehmen. Es sollen mit dem
erfindungsgemäßen Heilmittel vor allem Krebserkrankungen
bekämpft, aber auch gefährliche Blutergüsse o. Ödeme, z.B.
bei Rückgratverletzungen oder Gehirnblutungen beseitigt und Heilungen von Autoimmunerkrankungen erzielt werden.

Eine weitere Aufgabe der Erfindung ist es, mit den
gewonnenen Lymphzellen auch diejenigen in der Lymphflüssigkeit enthaltenen natürlichen Stoffe zusammen
mit den Lymphzellen auf den Patienten zu übertragen,
die zur Aktivierung der Immunreaktion dieser Lymphzellen dienen.

Diese Aufgabe wird dadurch gelöst, daß das erfindungsgemäße Heilmittel aus mit einem Filter von einer

- 3 -

Porenweite von 10 μm unter Druck von 0,3 bis 0,5 bar
gefilterter aus dem Ductus Thoracicus des Spendertieres entnommener Lymphe besteht.

Die Gewinnung der Lymphe aus dem Ductus Thoracicus
ist zwar bekannt, wie z. B. durch die in der oben
genannten US-PS beschriebene Ductus Thoracicus-
Trainage. Ferner ist sie erwähnt in Bier, Götze, Mota
und Dias da Silva: Immonologie, Berlin, Heidelberg,
New York, 1979 Seite 17 linke Spalte.

Diese Entnahme der Lymphe aus dem Milchbrustgang
lag aber deshalb nahe, weil er das einzige große
Lymphsammelgefäß ist, aus dem die Lymphe mit einer
Kanüle gut entnommen werden kann. Die Entnahme an
dieser Stelle des Lymphsystems erfolgt aber gemäß
der Erfindung deshalb, weil erkannt wurde, daß
dort Hormone der Nebenierenrinde und des Nebennierenmarks in die Lymphbahnen geleitet werden, die
eine Aktivierung der Lymphozyten bewirken (vgl.
Dr. Hans Selzer: "Die lymphogenen Erkrankungen
innerer Organe und des zentralen Nervensystems",
Verlag Laub GmbH & Co., Elztal, 1977, Seiten 89 + 90).
Es ist daher zweckmäßig dem erfindungsgemäßen
Heilmittel Extrakte von Nebennieren und Nebennierenrinde zuzusetzen. Dies ist vor allem bei Krebserkrankungen wichtig, weil bei diesen die Nebenniere
und insbesondere deren Rinde selbst erkrankt, so daß
durch deren Funktionsausfall der Lymphe des Patienten
keine Nebennierenhormone mehr zugeführt werden.

- 4 -

Die Verwendung eines Filters der genannten Porenweite
hat den Zweck, alle größeren Zellen, insbesondere
Epithel-, Ganglion- und Gefäßwandmuskelzellen und
deren Bruchstücke sowie sonstige grobmolekularen
Stoffe auszuschalten, die vom Immunsystem des
Empfängers als "fremd" erkannt werden können. In
der gewonnenen Flüssigkeit läßt sich mit der Sulfo-
Salicylsäure-Reaktion kein Eiweiß nachweisen. Sie
enthält neben den Antikörpern und Interferonen,
auf die es im wesentlichen hier nicht ankommt, nur
mehr die "kleinen" Lymphozyten, die im wesentlichen
noch nicht differenzierte oder noch nicht sensibilisierte T- und B-Zellen, aber nicht Plasmazellklonen
sind.

In der oben angegebenen Literaturstelle von Bier u.a.
Seite 11 rechte Spalte wird zwar gesagt, daß aus
dem Ductus Thoracicus durch Kanülierung kleine
Lymphozyten erhalten werden, die fähig waren, in bestrahlten Tieren die Antikörperbildung wieder herzustellen. Demnach wurden nicht differenzierte und nicht
sensibilisierte B-Lymphozyten im Wirtstier spezifisch
sensibilisiert, die dort proliferierten. Diese Versuche
wurden jedoch nur zum Zweck der Erforschung der
Physiologie des Immunsystems und nicht zu humantherapeutischen Zwecken unternommen, denen die
bereits genannten Vorurteile der Wahrscheinlichkeit
unerwünschter Immunreaktionen sowohl des Immunsystems
des Empfängers gegen die übertragenen Lymphozyten wie
umgekehrt dieser gegen das Immunsystem und die Gewebe
des Empfängers entgegenstanden.

- 5 -

Durch die Beschränkung der dem Patienten verabreichten
Lymphfraktion auf "kleine" Lymphozyten wird es möglich,
daß diese der Immunabwehrsituation des Wirtes entsprechend sensibilisiert werden, proliferieren und
die dort tatsächlich erforderlichen spezifischen
Globuline erzeugen und die insbesondere in der richtigen
Weise das Zellsystem des Wirtes als "selbst" und dessen
Antigene als "nicht selbst" erkennen und vor allem
nicht vom eigenen Immunsystems des Wirts als "fremd"
eliminiert werden.

Es ist notwendig, die Filterung der aus dem Ductus
Thoracicus entnommenen Lymphe möglichst schonend durchzuführen, um Beschädigungen der Lymphzellen, wie sie
z. B. beim Zentrifugieren eintreten, zu vermeiden. Es
ist daher zu empfehlen, nur für die kleinen Lymphozyten
durchlässige Membranfilter zu verwenden.

Es hat sich in überraschender Weise ergeben, daß die
übertragenen Lymphozyten in der Lage waren, nicht nur
bedrohliche Blutergüsse des Patienten aufzulösen,
sondern auch Krebsmetastasen, selbst in gefährlich
virulenten Formen wie Melanomen zur Abkapselung zu
bringen und auch Autoimmunkrankheiten zu heilen.

Das erfindungsgemäße Heilmittel ist eine lebende
Zellsubstanz und muß daher, obwohl einzelne Gruppen
der in ihm erhaltenen Zellen sehr viel längere
Lebenszeiten haben, binnen weniger Tage verbraucht
und bei 4°C gelagert werden.

- 6 -

Im folgenden werden einzelne Anwendungsbeispiele
beschrieben:

Der Anwendung der im nachfolgenden beschriebenen
Therapie auf Humanmedizin sind umfangreiche systematische
Versuche mit Tieren, insbesondere Kaninchen vorausgegangen, die zu reproduzierbaren überraschenden Heilerfolgen, insbesondere bei Krebserkrankungen geführt
hatten. Die drei nachstehend beschriebenen Fälle sind
zwar zunächst nur Einzelfälle, bei denen das erfindungsgemäße Heilmittel als letzte Möglichkeit eingesetzt
wurde. Es ergaben sich aber eindeutig den Tierversuchen
entsprechende Ergebnisse:

1. Der Patientin wurde im Alte von 20 Jahren ein
malignes Melanom im Bereich der Brustwirbelsäule
operativ entfernt. Drei Jahre später wurden zwei
Melanommetastasen im Gehirn durch Computer-Tomographie
festgestellt, weitere zwei Jahre später insgesamt
drei solche Metastasen. Da sich im folgenden Jahr bei
der bisherigen Behandlung keine Besserung zeigte,
wurde das erfindungsgemäße Heilmittel eingesetzt.
Es wurden im ganzen /6 paravertebrale intramuskuläre
Injektionen in einem Zeitraum von 38 Tagen gegeben,
die zu einer merklichen Besserung führten. Die Sehkraft war wieder normal, kein Doppelsehen oder Flimmern,
die bestehende Schlundlähmung war behoben, ebenso
Geruchs-, Geschmacks- und Gehörstörungen. Zeitweilige
Ataxien waren nicht mehr aufgetreten, die FWF-
Werte bewegten sich im Normalbereich, woraus geschlossen werden konnte, daß kein Gehirnödem bestand.

- 7 -

Die Patientin war so weit hergestellt, daß die Behandlung unterbrochen werden konnte. Bemerkenswert ist, daß weder Sprach- noch Gedächtnisstörungen noch Bein- oder Armlähmungen auftraten, wie sie bei Gehirntumoren zu erwarten sind. Der Tod der Patientin trat durch einen zerebralen Insult infolge nekrotischen Zerfalls im Bereich der Hirntumoren sehr bald nach der letzten Behandlung ein. Der Obduktionsbefund zeigte, daß, obwohl drei Gehirnmetastasen vorhanden waren mit Ausnahme von zwei Metastasen in der Leber in den übrigen Organen keine einzige gefunden werden konnte. Die eine Metastase im Leberparenchym war erbsengroß, die andere haselnußgroß. Sie ergaben folgenden Befund:

Leber: Zentral ausgedehnt nekrotisch zerfallene Metastasen des seit Jahren bekannten malignen Melanoms. Melaninhaltige und intakte Tumorzellen sind nur noch in einem schmalen Randbezirk erkennbar. Die Metastasen sind durch einen teils breiteren, teils schmäleren Bindegewebsstreifen gegenüber dem umgebenden Leberparenchym abgegrenzt. Die Knoten sind in sich geschlossen, ein diffuses und grenzenloses infiltrierendes Wachstum ist nicht erkennbar. Leichte, grob- bis mitteltropfige, vornehmlich läppchenzentral etablierte Leberepithelverfettung, wenige bis mäßig reichlich vorhandene sog. Kollapsstraßen des Leberparenchyms.

Die Lymphknoten zeigten eine Vergrößerung mit deutlicher Hyperämie, was auf eine erhöhte Aktivität des Lymphsystems schließen läßt.

- 8 -

2. Der Patient litt in einem sehr fortgeschrittenen
Stadium an amyotrophischer Lateralsklerose, deren
Ursache unbekannt ist, die als unheilbar gilt und
in zwei bis vier Jahren zum Tod durch Atemlähmung
führt. Die Beweglichkeit der Beine und des Schultergürtels waren bereits durch Muskellähmungen sehr eingeschränkt, daneben bestanden bulbärparalytische
Beschwerden. Die Beine konnten nur bis zu 60° angehoben werden. Die Arme hingen schlaff herunter,
der Patient mußte gefüttert werden. Schreiben war
unmöglich. In einer Zeit von nicht ganz drei Monaten
wurden im ganzen 10 Injektionen des erfindungsgemäßen
Heilmittels in die vertebrale Muskulatur in der
Höhe des Halsmarkes und in die Clutäusmuskulatur in
der Höhe des lumbalen Marks gegeben. Es trat eine
Stärkung der bereits atrophischen Muskulatur und
bei besserer Beweglichkeit eine gute Durchblutung
der Arme und Beine ein. Die Sprachstörungen sind zurückgegangen und die Sprache ist bedeutend deutlicher.
Verschlucken, das vorher sehr häufig war, tritt nur
noch selten auf. Die Schleimbildung in der Lunge
ist gut zurückgegangen und kann leicht beseitigt
werden, was vorher nicht möglich war und es besteht
ein guter Allgemeinzustand und gute Aussichten auf
weitere Besserung.

3. Der Patient leidet an Multipliskerose des II. bis
III. Grades. Infolge einer Kriegsverletzung bestand
eine chronische großflächige (ca. 20 x 10 cm) Entzündung der rechten unteren Bauchmuskulatur mit
drei Darmfisteln, die trotz 15-maliger Operation innerhalb von 38 Jahren nicht beseitigt werden konnten.

- 9 -

Nach einer Behandlung mit 10 intramuskulären Injektionen
des erfindungsgemäßen Heilmittels sind folgende Ergebnisse eingetreten:

1. Die Blutsenkung hat sich innerhalb von
   vier Wochen, in der ersten Woche mit
   50/100, in der zweiten Woche mit 70/125,
   in der dritten Woche mit 75/130*) auf
   17/46 in der vierten Woche gebessert.

2. Die Gefühlsstörung, die in der rechten
   Hand vorhanden war, ist verschwunden.

3. Die Schwäche der Beinmuskulatur hat sich
   wesentlich gebessert.

4. Keine Gleichgewichtsstörungen oder Unsicherheiten beim Treppensteigen.

5. Er kann jetzt 3 - 4 km auf der Straße gehen.

6. Die Darmfisteln sind innerhalb von vier
   Wochen vollkommen abgeheilt.

Zu bemerken ist, daß in allen den drei beschriebenen
Fällen keine Immunabwehrreaktionen des Patienten
gegen das erfindungsgemäße Heilmittel eintraten, insbesondere keine allergischen oder entzündlichen
Reaktionen. Daß im ersten der drei genannten Fälle die
bereits ausgebreiteten Hirntumoren durch die Behandlung
nicht angegriffen werden konnten, entspricht der Erfahrung, daß die Immunabwehr nur gegen entstehende
Tumoren wirksam sein kann. Der beschriebene Fall hat
jedoch bewiesen, daß eine passive Immunisierung durch
Spenderlymphozyten möglich ist, wobei mit dem erfindungsgemäßen Heilmittel die bekannten Schwierigkeiten,
immunologisch reaktive spezifizierte Zellen überhaupt
und dann in ausreichendem Maß zu erhalten, durch die

(* nach Einsetzen des erfindungsgemäßen Heilmittels

- 10 -

Verwendung noch nicht sensibilisierter bzw. nicht spezifizierter Zellen umgangen ist.Dabei kann das bei Autoimmunerkrankungen verschobene immunologische Gleichgewicht durch Ausgleich des Verhältnisses zwischen Suppressorzellen und autoallergischen Zellen (verbotenen Klonen) wiederhergestellt werden, wie dies Beispiel 2 und 3 nahelegt.

Patentansprüche:


1. Heilmittel aus Lymphe von Säugetieren,
dadurch gekennzeichnet, daß es aus mit einem Filter
von einer Porenweite von höchstens 10 µm unter Druck
von 0,3 bis 0,5 bar gefilterter aus dem Ductus
Thoracicus eines Spendertieres entnommener Lymphe besteht.


2. Heilmittel nach Anspruch 1,
dadurch gekennzeichnet, daß ihm Extrakte von Nebenniere und Nebennierenrinde der gleichen oder gleichartigen Spendertiere zugesetzt sind.


3. Heilmittel nach Anspruch 1, 2,
dadurch gekennzeichnet, daß der Filter ein nur für
kleine Lymphozyten durchlässiger Membranfilter ist.

ANKKONTO: DEUTSCHE BANK AG MÜNCHEN 22/26 843 (BLZ 700 700 10) · POSTSCHECKKONTO: MÜNCHEN 1999 94-803
ANDERKONTO: DEUTSCHE BANK AG MÜNCHEN 22/26 850 (BLZ 700 700 10)

0068042
Nummer der Anmeldung

**Europäisches Patentamt**

**EUROPÄISCHER RECHERCHENBERICHT**

EP 81 10 5110

| Kategorie | Kennzeichnung des Dokuments mit Angabe, soweit erforderlich, der maßgeblichen Teile | betrifft Anspruch |
|---|---|---|
| Y | CHEMICAL ABSTRACTS, Band 94, Nr. 8, 23. Februar 1981, Zu-sammenfassung Nr. 43697r, Seiten 259,260, COLUMBUS OHIO (US) & JP - A - 80 135 750 (ASAHI CHEMICAL INDUSTRY CO., LTD.) (22.10.1980)  * Zusammenfassung * | 1,3 |
| Y | CHEMICAL ABSTRACTS, Band 94, Nr. 8, 23. Februar 1981, Zu-sammenfassung Nr. 43698s, Seite 260 COLUMBUS OHIO (US) & JP - A - 80 135 749 (ASAHI CHEMICAL INDUSTRY CO., LTD.) ( 22.10.1980)  * Zusammenfassung * | 1,3 |
| Y | BIOLOGICAL ABSTRACTS, Band 56, Nr. 1, 1973, Zusammenfassung Nr. 2543, Seite 256 PHILADELPHIA, PENNSYLVANIA (US) & Clin exp. immunol 12(2):255-262 R.J. SHARBAUGH et al.: "The efficacy of closed-circuit extracorporeal filtration of thoracic duct lymph as a means of lymphocyte depletion".  * Zusammenfassung * | 1,3 |

./..

**EINSCHLÄGIGE DOKUMENTE**

**KLASSIFIKATION DER ANMELDUNG (Int. Cl.³)**

A 61 K 35/26//
A 61 K 35/26
35/23

**RECHERCHIERTE SACHGEBIETE (Int. Cl.³)**

A 61 K

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde lie-gende Theorien oder Grund-sätze
E: älteres Patentdokument, das jedoch erst am oder nach dem Anmeldedatum veröffentlicht worden ist
D: in der Anmeldung angeführtes Dokument
L: aus andern Gründen ange-führtes Dokument

&: Mitglied der gleichen Patent-familie, übereinstimmendes Dokument

X | Der vorliegende Recherchenbericht wurde für alle Patentansprüche erstellt.

| Recherchenort | Abschlußdatum der Recherche | Prüfer |
|---|---|---|
| Den Haag | 22.02.1982 | MARIE |

EPA form 1503.1   06.78

Europäisches
Patentamt

EUROPÄISCHER RECHERCHENBERICHT

0068042
Nummer der Anmeldung

EP 81 10 5110
-2-

| Kategorie | Kennzeichnung des Dokuments mit Angabe, soweit erforderlich, der Maßgeblichen Teile | betrifft Anspruch | KLASSIFIKATION DER ANMELDUNG (Int. Cl.³) |
|---|---|---|---|
| | **EINSCHLÄGIGE DOKUMENTE** | | |
| Y | BIOLOGICAL ABSTRACTS, Band 64, Nr. 8, 1977, Zusammenfassung Nr. 20878, Seite 2045 PHILADEPHIA, PENNSYLVANIA (US) & J. Surg. Oncol 9(2): 155-162 J.A. MAJESKI et al.: "Architectural alteration of lymphatic tissue produced by extracorporeal thoracic duct filtration". * Zusammenfassung * | 1,3 | |
| Y | IVAN M. ROITT: ESSENTIAL IMMUNOLOGY, 4. Auflage, 1980 Blackwell Scientific Publications Oxford, London, Edinburgh, Boston und Melbourne Seiten 51,52 * Seiten 51,52 * | 1,3 | RECHERCHIERTE SACHGEBIETE (Int. Cl.³) |
| D/A | US - A - 3 719 182 (SAMUEL ROSE) * das ganze Dokument * | 1,3 | |